(19) Europäisches Patentamt
European Patent Office
Office européen des brevets

(11) **EP 4 183 404 A1**

(12) **EUROPEAN PATENT APPLICATION**
published in accordance with Art. 153(4) EPC

(43) Date of publication:
**24.05.2023   Bulletin 2023/21**

(21) Application number: **21845375.1**

(22) Date of filing: **16.07.2021**

(51) International Patent Classification (IPC):
*A61K 36/23* (2006.01)       *A61P 17/14* (2006.01)
*A61K 8/9789* (2017.01)       *A61Q 7/00* (2006.01)

(86) International application number:
**PCT/KR2021/009164**

(87) International publication number:
**WO 2022/019573 (27.01.2022 Gazette 2022/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **20.07.2020   KR 20200089738**

(71) Applicants:
 • **Daebong LS Co., Ltd.**
   **Incheon 21697 (KR)**
 • **UCL Co. Ltd.**
   **Jeju-si, Jeju-do 63038 (KR)**

(72) Inventors:
 • **YEOM, Hyun-Sook**
   **Jeju-si, Jeju-do 63293 (KR)**

 • **KIM, Ji-Hye**
   **Seogwipo-si, Jeju-do 63599 (KR)**
 • **OH, Won-Bo**
   **Seogwipo-si, Jeju-do 63623 (KR)**
 • **LEE, Hye-Ja**
   **Seogwipo-si, Jeju-do 63571 (KR)**
 • **PARK, Jin-Oh**
   **Seoul 06276 (KR)**
 • **MOON, Ji-Young**
   **Jeju-si, Jeju-do 63098 (KR)**
 • **KIM, Jeong-Mi**
   **Jeju-si, Jeju-do 63137 (KR)**
 • **PARK, Byoung-Kwon**
   **Incheon 22002 (KR)**
 • **LEE, Ji-Won**
   **Seoul 06276 (KR)**

(74) Representative: **Petraz, Gilberto Luigi et al
GLP S.r.l.
Viale Europa Unita, 171
33100 Udine (IT)**

(54) **COMPOSITION FOR PREVENTING HAIR LOSS OR PROMOTING HAIR GROWTH COMPRISING EXTRACT OF ABOVE-GROUND PART OF CARROTS**

(57)   The present disclosure relates to a composition that is capable of preventing hair loss and promoting hair growth by comprising the extract of the above-ground part of carrots. The composition comprising the extract of the above-ground part of carrots of the present disclosure significantly increases the viability of dermal papilla cells and can inhibit the expression of $5\alpha$-reductase, thereby exhibiting excellent effects in preventing hair loss and promoting hair growth.

EP 4 183 404 A1

## Description

### Technical Field

The present disclosure relates to a composition that can prevent hair loss and promote hair growth, including an extract of the above-ground part of carrots. This application claims priority based on Korean Patent Application No. 10-2020-0089738 filed on July 20, 2020, and all contents described in the specification and drawings of the application are incorporated herein by reference.

### Background Art

Hair loss refers to an increase in abnormally falling hair due to a higher proportion of hair growing in the follicles in the degenerative or resting phase than the hair growing in the follicles in the growth phase during the hair follicle growth cycle.

The causes of such hair loss include a decreased function or inhibition of proliferation of dermal papilla cells and geminal matrix cells involved in hair cycle regulation, excessive action of male hormones, decrease in blood flow to hair follicles, excessive sebum secretion, decreased scalp function due to peroxide and bacteria, genetic factors, aging, and neurosis due to stress, and various causes are known to act alone or in combination.

Recently, the incidence of alopecia has been increasing among modern people due to environmental factors such as air pollution and mental stress, and the age of occurrence is getting younger. Such alopecia may cause serious stress to the parties and may interfere with interpersonal relationships, so interest in hair loss treatment and prevention is increasing.

Various external preparations are used to treat hair loss, but the most widely used is a minoxidil-containing formulation of Upzon, approved by the US FDA. Recently, Propecia, which is a main component of finasteride that has an effect of inhibiting 5 alpha-reducing enzymes of Type 2, released by Merck, has been used. However, in the case of minoxidil, there are problems such as itching and irritation, and in the case of finasteride, side effects such as reduced sexual desire and erectile dysfunction occur, and if it is stopped, the hair returns to its original state within two months, and women cannot take it because there is a risk of giving birth to deformed babies.

In addition, studies based on natural extracts with few side effects, such as extracts of Cnidium officinale, Phellinus linteus, Lonicera japonica, Chamomile, and other natural extracts of Polygonum multiflorum, Acorus calamus L., pomegranate, ginkgo leaf are being conducted. When these hair growth agents are actually used in clinical practice, the effect on the scalp is not so excellent.

In Korea Patent No. 10-2065395, disclosed is a hair growth agent composition containing natural extracts such as camellia flower, red pepper seed, eggplant, aloe, Dendranthema zawadskii var. latilobum, carrot, spinach, Psoralea corylifolia L., Mori Cortex Radicis, ginger, lotus root, dandelion, Crinum asiaticum var. japonicum Baker, safflower, green tea, Houttuynia cordata Thunberg, turmeric, ginkgo leaf, Acorus gramineus Soland, and research to discover a hair loss agent or a hair growth agent made of a natural material that has such an excellent effect and has few side effects is continuously required (Patent Literature 1) .

[Related art literature]

[Patent Literature]

(Patent Literature 1) Korean Patent No. 10-2065395

### Disclosure

### Technical Problem

Accordingly, the present inventors made research efforts to develop a composition that exhibits an excellent effect in preventing hair loss or promoting hair growth. As a result, the present disclosure was completed by confirming that the extract of the above-ground part of carrots contains a natural substance and has excellent effects of preventing hair loss and promoting hair growth while having few side effects.

Accordingly, an objective of the present disclosure is to provide a composition for preventing hair loss or promoting hair growth that includes an extract of the above-ground part of carrots.

**Technical Solution**

The present disclosure provides a composition for preventing hair loss or promoting hair growth, including an extract of the above-ground part of carrots.

The above-ground part of carrots may be leaves, flowers, stems, buds, or a mixture thereof.

The above-mentioned extract of the above-ground part of carrots may be obtained by extracting the above-ground part of carrots using water, C1 to C4 alcohol, or a mixture thereof as a solvent.

The extract of the above-ground part of carrots may be included in a concentration of 0.001% to 0.02% by weight.

The composition for preventing hair loss or promoting hair growth may inhibit $5\alpha$-reductase.

The present disclosure also provides a pharmaceutical composition for external use on the skin, including an extract of the above-ground part of carrots extracted at room temperature by putting the above-ground part of carrots in a mixed solvent in which water and ethanol are mixed in a weight ratio of 1:9 to 5:5.

The present disclosure also provides a cosmetic composition, including an extract of the above-ground part of carrots extracted at room temperature by putting the above-ground part of carrots in a mixed solvent in which water and ethanol are mixed in a weight ratio of 1:9 to 5:5.

**Advantageous Effects**

The composition, including the extract of the above-ground part of carrots of the present disclosure, can significantly increase the viability of dermal papilla cells and inhibit the expression of $5\alpha$-reductase, thereby showing an excellent effect in preventing hair loss and promoting hair growth.

**Best Mode**

Hereinafter, the present disclosure will be described in detail. Prior to this, the terms or words used in the present specification and claims should not be construed as being limited to conventional or dictionary meanings. An inventor should be interpreted as a meaning and concept consistent with the technical idea of the present invention based on the principle that the concept of terms can be appropriately defined in order to describe his or her own invention in the best way. Therefore, since the configurations described in the embodiments described herein are only the most preferred embodiments of the present disclosure and do not represent all the technical ideas of the present disclosure, it should be understood that there may be various equivalents and modifications that may replace them at the time of the present application.

The composition for preventing hair loss or promoting hair growth of the present disclosure includes an extract of the above-ground part of carrots (*Daucus carota*).

The above-ground part of carrots may be leaves, flowers, stems, buds, or a mixture thereof, but the composition may preferably include leaves and stems extracts.

The extract of the above-ground part of carrots may be obtained by extracting the above-ground part of carrots by using water, C1 to C4 alcohol, or a mixture thereof as a solvent, but preferably, a mixture of water and ethanol as a solvent may be used. More preferably, the extract is preferable to use a mixed solvent in which water and ethanol are mixed in a weight ratio of 1:9 to 5:5 as a solvent.

The extract of the above-ground part of carrots may be obtained by extracting the above-ground part of carrots at room temperature, preferably, at a temperature range of 10°C to 30°C, and more preferably, 15°C to 25°C.

The extract of the above-ground part of carrots may be included in a concentration of 0.001% to 1% by weight in the composition, preferably 0.005% to 0.05% by weight, and most preferably, 0.01% to 0.02% by weight.

The composition of the present disclosure may significantly increase the viability of dermal papilla cells and inhibit the expression of $5\alpha$-reductase.

Human dermal papilla cells exist at the base of the follicle, and dermal papilla cells are responsible for hair growth and hair follicle cycle control by supplying oxygen and nutrition to the cells that make up the follicle. Therefore, when the proliferation of dermal papilla cells is promoted, hair becomes healthy, hair growth is promoted, and hair loss can be prevented. Because hair growth proceeds as the epithelial cells surrounding the dermal papilla divide to form a hair shaft, the dermal papilla cells play an important role in regulating the division of epithelial cells. In addition, in androgenetic alopecia, the site where male hormones act on hair follicles is also the dermal papilla, and dermal papilla cells play a very important role in hair growth.

In addition, $5\alpha$-reductase is known to have type 1 and type 2 isoenzymes. $5\alpha$-reductase-1 of type 1 is mainly distributed in a dermal papilla cell, sebaceous glands, and keratinocytes of epidermis and hair follicles, and $5\alpha$-reductase-2 of type 2 is mainly distributed in the prostate, genital skin, seminal vesicles, and epididymis. $5\alpha$-reductase is an enzyme that acts with testosterone to produce dihydrotestosterone (DHT), a major cause of hair loss, and the reduction of $5\alpha$-reductase-1 gene expression in human dermal papilla cells is known to be involved in hair loss prevention.

The composition is a pharmaceutical composition for external application to the skin and may be a cream, gel, patch, spray, ointment, plaster, lotion, liniment agent, paste, or cataplasma formulation, but is not limited thereto.

In addition, the composition is a cosmetic composition and may be a hair tonic, hair conditioner, hair essence, hair lotion, hair nutrition lotion, hair shampoo, hair conditioner, hair treatment, hair cream, hair nutrition cream, hair moisture cream, hair massage cream, hair wax, hair aerosol, hair pack, hair nourishment pack, hair soap, hair cleansing foam, hair oil, hair drying agent, hair preservative, hair dye, hair wave agent, hair bleach, hair gel, hair glaze, hairdressing agent, hair lacquer, hair moisturizer, hair mousse, or hair spray formulation, but is not limited thereto.

The composition may include ingredients commonly used in cosmetic compositions in addition to the extract of the above-ground part of carrots. For example, the composition may include adjuvants commonly used in the field of cosmetology or dermatology, such as fatty substance, organic solvent, solubilizer, thickener, gelling agent, emollient, antioxidant, suspending agent, stabilizer, foaming agent, fragrance, surfactant, water, an ionic or nonionic emulsifier, a filler, a sequestering agent, a chelating agent, a preservative, vitamins, a blocking agent, a wetting agent, essential oil, a dye, a pigment, a hydrophilic or lipophilic active agent, a lipid vesicle, or any other ingredients commonly used in cosmetics. The adjuvant is introduced in an amount generally used in the field of cosmetology or dermatology.

Hereinafter, examples and experimental examples will be described in detail to describe the present disclosure in detail. However, the embodiments according to the present disclosure may be modified in various other forms, and the scope of the present disclosure should not be construed as being limited to the embodiments described below. The embodiments of the present disclosure are provided to more completely explain the present disclosure to those of ordinary skilled in the art.

Example: preparation of the extract of the above-ground part of carrots

Carrot leaves were washed with distilled water, dried, and pulverized with a blender to obtain a fine powder sample. Next, a solvent in which ethanol and purified water were mixed at a weight ratio of 7: 3 was added in an amount of about 20 times the volume of the fine powder sample and then extracted once at 25°C. After the extraction was performed, the resulting filtrate was filtered through a 400 mesh filter, concentrated using a decompression concentrator, and dissolved in purified water to a concentration of 5 mg/mL to prepare a stock solution. Then, the stock solution was diluted with purified water, and the working solution adjusted to individual concentration was used for the following test.

Test Example 1: cell culture

Human follicle dermal papilla cell culture

Human follicle dermal papilla cells were purchased from PromoCell Co., and a human follicle dermal papilla cell growth medium containing fetal calf serum, bovine pituitary extract, basic fibroblast growth factor and insulin was used and cultured at 37°C and 5% $CO_2$ incubator.

MCTT HCE™ culture

MCTT HCE™ is a human corneal model (organization) that reproduces the corneal epithelium using human corneal epithelial cells, and the tissue manufactured by Korea Bio-solution was purchased and used. The tissue placed on the agarose gel was transferred to a 24-well plate and pre-cultured at 37°C and 5% $CO_2$ incubator.

Test Example 2: Cytotoxicity evaluation

MTT assay

MTT assay is a representative method for measuring cell viability using the principle that MTT (3-(4,5-dimethylthiazol-2-yl-2,5-diphenyltetrazolium bromide) reacts with dehydrogenase of living cells to generate purple formazans.

In order to check the cytotoxicity, $1 \times 10^4$ cells/mL of human dermal papilla cell were dispensed into a 48 well plate, and when the amount reached 80% or more, the extract of the above Example was treated with the concentration shown in Table 1 below and cultured for 24 hours. Then, 200 μL of a 0.5 mg/mL solution of MTT diluted in the medium were added and cultured at 37°C for 4 hours. After removing the solution, adding 200 μL of DMSO, and transferring 100 μL to a 96 well plate, absorbance was measured at 585 nm using a microplate reader. The average absorbance value for each sample group was obtained, and the cell viability was evaluated by comparing the average absorbance value with the absorbance value of the control group.

[Table 1]

| Division | Cytotoxicity | |
|---|---|---|
| | Treatment concentration (wt%) | Cell viability (%) |
| Untreated group | - | 100.000 |
| Example | 0.00005 | 100.496 |
| | 0.0005 | 104.311 |
| | 0.005 | 105.798 |
| | 0.01 | 110.631 |
| | 0.02 | 115.396 |
| | 0.05 | 14.222 |

As shown in Table 1, it was confirmed that cytotoxicity did not appear when the extract of the above Example was treated at 0.02% by weight or less and that cell viability rapidly decreased due to strong cell toxicity when the concentration of the extract was more than 0.05% by weight.

Test Example 3: dermal papilla cell viability

When the cultured human dermal papilla cells were dispensed in a 48 well plate at $1 \times 10^4$ cell/mL, and when the amount reached 80% or more, the extract of Example was treated at the concentrations shown in Table 2 below. The positive control group was treated with minoxidil at concentrations of 10 and 50 $\mu$M. After culturing the cells for 24, 48, and 72 hours, 200 $\mu$L of a 0.5 mg/mL solution of MTT diluted in the medium were added and cultured at 37°C for 4 hours. After removing the solution, adding 200 $\mu$L of DMSO, and transferring 100 $\mu$L to a 96 well plate, absorbance was measured at 585 nm using a microplate reader. The average absorbance value for each sample group was obtained, and the cell viability was evaluated by comparing the average absorbance value with the absorbance value of the control group.

[Table 2]

| Division | | 24h | 48h | 72h |
|---|---|---|---|---|
| Positive control group | 10 $\mu$M | 104.056 | 107.385 | 115.000 |
| | 50 $\mu$M | 107.407 | 116.168 | 124.815 |
| Example | 0% by weight | 100.000 | 100.000 | 100.000 |
| | 0.005% by weight | 103.704[#] | 109.980[#] | 118.519[#] |
| | 0.01% by weight | 116.931[#] | 120.559[#] | 128.519[#] |
| | 0.02% by weight | 121.693[#] | 125.749[#] | 135.185[#] |

As shown in Table 2, when the extract of Example was treated with 0.01 to 0.02% by weight, it was confirmed that the viability of human dermal papilla cells was superior to that of minoxidil, a positive control group was increased.
In addition, in order to confirm the effect according to the solvent composition, a water solvent (Comparative Example 1) and a mixed solvent (Comparative Example 2) in which water and ethanol were mixed in a weight ratio of 7:3 were used to prepare an extract of the above-ground part of carrots. After that, the viability test for human dermal papilla cell was performed in the same manner as above.

[Table 3]

| | Concentration (wt%) | 24H | 48H | 72H |
|---|---|---|---|---|
| Purified water extraction (room temperature) | 0.005 | 90.585 | 93.923 | 91.023 |
| | 0.01 | 94.047 | 95.844 | 91.377 |
| | 0.02 | 100.142 | 96.198 | 90.574 |

[Table 4]

| Concentration (% by weight) | Comparative Example 1 / Example Ratio (%) | | |
|---|---|---|---|
| | 24H | 48H | 72H |
| 0.005 | 87.3 | 85.4 | 76.8 |
| 0.01 | 80.4 | 79.5 | 71.1 |
| 0.02 | 82.3 | 76.5 | 67.0 |

As shown in Table 3 above, when extracted with purified water, the viability of human dermal papilla cells is the same as 100% or less, so it can be confirmed that the extract of the above-ground part of carrots with purified water does not substantially improve hair loss.

In addition, the ratio (%) of Comparative Example 1/Example of the 9 cells of Table 4 is, on average, about 78.5%. It was confirmed that the viability of human dermal papilla cells was significantly higher when extracted with 70% by weight of ethanol than when extracted with a water solvent.

[Table 5]

| 30% by weight of ethanol extraction (room temperature) | Concentration (% by weight) | 24H | 48H | 72H |
|---|---|---|---|---|
| | 0.005 | 99.48 | 100.458 | 95.62 |
| | 0.01 | 100.832 | 96.081 | 91.573 |
| | 0.02 | 101.334 | 95.522 | 90.257 |

[Table 6]

| Concentration (% by weight) | Comparative Example 2 / Example Ratio (%) | | |
|---|---|---|---|
| | 24H | 48H | 72H |
| 0.005 | 95.9 | 91.3 | 80.7 |
| 0.01 | 86.2 | 79.7 | 71.3 |
| 0.02 | 83.3 | 76.0 | 66.8 |

The ratio (%) of Comparative Example 2/Example of the 9 cells of Table 6 was about 81.2% on average. It was confirmed that the viability of human dermal papilla cells was significantly higher when extracted with 70% by weight of ethanol than when extracted with 30% by weight ethanol.

Next, in order to confirm the effect according to the extraction temperature, the viability of the human dermal papilla cells was measured after obtaining the extract by heating it to 80°C while using 70% by weight of ethanol (Comparative Example 3).

[Table 7]

| 70% by weight of ethanol extraction (heated to 80°C) | Concentration (% by weight) | 24H | 48H | 72H |
|---|---|---|---|---|
| | 0.005 | 108.195 | 111.45 | 102.377 |
| | 0.01 | 111.175 | 112.723 | 106.796 |
| | 0.02 | 113.063 | 112.265 | 116.618 |

[Table 8]

| Concentration (% by weight) | Comparative Example 3 / Example Ratio (%) | | |
|---|---|---|---|
| | 24H | 48H | 72H |
| 0.005 | 104.3 | 101.3 | 86.4 |
| 0.01 | 95.1 | 93.5 | 83.1 |
| 0.02 | 92.9 | 89.3 | 86.3 |

In the case of the present disclosure, as shown in Table 1, while the viability of human dermal papilla cell increases over time, the extracts obtained by heating in Comparative Example 3 of Table 7 showed a tendency to decrease over time. In addition, in Table 8 comparing the two results, it was confirmed that the heated extract showed only an effect of about 85% level after 72 hours when compared with the extract at room temperature.

Therefore, it was confirmed that the extract of the above Example could exhibit the effect of preventing hair loss and promoting hair growth by improving the viability of dermal papilla cells.

Test Example 4: Evaluation of gene expression of $5\alpha$-reductase-1

Real-time polymerase chain reaction (qRT-PCR)

This test is a method to confirm the gene expression level of $5\alpha$-reductase-1 using a real-time polymerase chain reaction. RNA extracted from cells is used to make cDNA by reverse transcriptase and amplified through a polymerase chain reaction so that the expression level of the corresponding gene can be compared in real-time and relatively.

When the cultured human dermal papilla cells were dispensed in a 6-well plate at $2 \times 10^5$ cell/mL, and the amount reached 90% or more, the extract of Example was treated in each well and cultured for 24 hours. Thereafter, cells were suspended by treatment with 0.05% Trypsin-EDTA (Gibco), and total RNA in the cells was extracted using an RNA extraction kit (RNeasy mini kit, Qiagen). cDNA was synthesized from the extracted total RNA using RNA to cDNA EcoDryTM Premix (Oligo dT) (Clontech). A real-time polymerase chain reaction was performed using the synthesized cDNA, Taqman Fast Advanced Master Mix (Applied Biosystems), and Taqman primer ($5\alpha$-reductase-1: Hs00971645_g1, Applied Biosystems) of each target. For the relative quantitative analysis of each gene, GAPDH (Hs02786624_g1, Applied Biosystems), a housekeeping gene, was used.

During cDNA amplification, the amplification amount was checked in real-time to obtain a threshold cycle (CT), which is an intersection between the amplification curve and the threshold line, and a relative quantitative value (RQ) was obtained based on this value to confirm the relative mRNA expression of the target gene. The RQ was calculated by the following Formula.

[Formula 1]

$$RQ = 2^{-\Delta\Delta C_T}$$

$$\Delta\Delta C_T = \Delta C_T \text{ (treatment)} - \Delta C_T \text{ (control)}$$

$$\Delta C_T = C_T \text{ (target gene)} - C_T \text{ (housekeeping gene)}$$

- Treatment (test group): Example treatment
- Control (control group): negative control group
- target gene: $5\alpha$-reductase-1
- housekeeping gene: GAPDH

[Table 9]

|  |  | Negative control group | Example extract (0.005% by weight) |
|---|---|---|---|
| $5\alpha$-reductase-1 gene expression level (RQ) | Average | $1.000 \pm 0.062$ | $0.672 \pm 0.018$ |
| Significance probability (p-value) for negative control group |  | - | $<0.05^{\#}$ |

As shown in Table 9, it was confirmed that the extract of the above-ground part of carrots of Example, suppressed the expression of the $5\alpha$-reductase-1 gene (mRNA) compared to the negative control group.

**Claims**

1. A composition for preventing hair loss or promoting hair growth, the composition comprising an extract of above-ground part of carrots.

2. The composition of claim 1, wherein the above-ground part of carrots is a leaf, a flower, a stem, a sprout, or a mixture thereof.

3. The composition of claim 1, wherein the extract of the above-ground part of carrots is extracted using water, C1 to C4 alcohol, or a mixture thereof as a solvent.

4. The composition of claim 1, wherein the extract of the above-ground part of carrots is contained in a concentration of 0.001% to 0.02% by weight.

5. The composition of claim 1, inhibits $5\alpha$-reductase.

6. A pharmaceutical composition for external use for skin, the composition comprising an extract of an above-ground part of carrots, wherein the above-ground part of carrots is extracted at room temperature by putting the above-ground part of carrots in a mixed solvent in which water and ethanol are mixed in a weight ratio of 1:9 to 5:5.

7. A cosmetic composition comprising an extract of an above-ground part of carrots, wherein the above-ground part of carrots is extracted at room temperature by putting the above-ground part of carrots in a mixed solvent in which water and ethanol are mixed in a weight ratio of 1:9 to 5:5.

## INTERNATIONAL SEARCH REPORT

| International application No. |
| --- |
| **PCT/KR2021/009164** |

| A. | CLASSIFICATION OF SUBJECT MATTER |
| --- | --- |

**A61K 36/23**(2006.01)i; **A61P 17/14**(2006.01)i; **A61K 8/9789**(2017.01)i; **A61Q 7/00**(2006.01)i

According to International Patent Classification (IPC) or to both national classification and IPC

| B. | FIELDS SEARCHED |
| --- | --- |

Minimum documentation searched (classification system followed by classification symbols)

A61K 36/23(2006.01); A61K 8/97(2006.01); A61K 8/9789(2017.01); A61Q 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 당근(carrot), 탈모(alopecia), 발모(hair growth)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 61-093109 A (AKIO, F.) 12 May 1986 (1986-05-12)<br>See claim 1; page 2; and the examples. | 1-7 |
| A | KR 10-2065395 B1 (JG CO., LTD.) 14 January 2020 (2020-01-14)<br>See claims 1 and 4. | 1-7 |
| A | KR 10-0668878 B1 (10-0668878) 12 January 2007 (2007-01-12)<br>See claims 1-8. | 1-7 |
| A | KR 10-2004-0032701 A (KIM, Hun Tae) 17 April 2004 (2004-04-17)<br>See entire document. | 1-7 |
| PX | KR 10-2255264 B1 (DAEBONG LS CO., LTD. et al.) 24 May 2021 (2021-05-24)<br>See claims 1-2 and 5-7.<br>*Published patent of a priority application of the present PCT application. | 1 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 November 2021** | **15 November 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

| International application No. |
| --- |
| **PCT/KR2021/009164** |

| Patent document cited in search report | | | Publication date (day/month/year) | Patent family member(s) | Publication date (day/month/year) |
| --- | --- | --- | --- | --- | --- |
| JP | 61-093109 | A | 12 May 1986 | None | |
| KR | 10-2065395 | B1 | 14 January 2020 | KR 10-2019-0010814    A | 31 January 2019 |
| KR | 10-0668878 | B1 | 12 January 2007 | None | |
| KR | 10-2004-0032701 | A | 17 April 2004 | None | |
| KR | 10-2255264 | B1 | 24 May 2021 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200089738
- KR 102065395